(19) Europäisches Patentamt
European Patent Office
Office européen des brevets

(11) **EP 4 321 487 A1**

(12) **EUROPEAN PATENT APPLICATION**
published in accordance with Art. 153(4) EPC

(43) Date of publication:
**14.02.2024 Bulletin 2024/07**

(21) Application number: **22784320.8**

(22) Date of filing: **02.02.2022**

(51) International Patent Classification (IPC):
*C02F 1/44* (2023.01)     *B01D 61/02* (2006.01)
*B01D 61/14* (2006.01)     *B01D 61/58* (2006.01)
*B01D 65/08* (2006.01)     *C02F 1/58* (2023.01)
*C02F 1/60* (2023.01)     *C02F 1/62* (2023.01)
*C02F 1/64* (2023.01)     *C02F 3/12* (2023.01)
*C02F 3/30* (2023.01)     *C02F 3/34* (2023.01)
*C12P 1/00* (2006.01)     *C12P 7/62* (2022.01)

(52) Cooperative Patent Classification (CPC):
**B01D 61/02; B01D 61/14; B01D 61/58;
B01D 65/08; C02F 1/44; C02F 1/58; C02F 1/60;
C02F 1/62; C02F 1/64; C02F 3/12; C02F 3/30;
C02F 3/34; C12P 1/00; C12P 7/62**

(86) International application number:
**PCT/JP2022/003969**

(87) International publication number:
**WO 2022/215335 (13.10.2022 Gazette 2022/41)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **09.04.2021   JP 2021066525**

(71) Applicant: **Kaneka Corporation
Osaka-shi, Osaka 530-8288 (JP)**

(72) Inventors:
• **FUKUMOTO, Asuka
Takasago-shi, Hyogo 676-8688 (JP)**
• **HIRANO, Masaru
Takasago-shi, Hyogo 676-8688 (JP)**

(74) Representative: **Vossius & Partner
Patentanwälte Rechtsanwälte mbB
Siebertstrasse 3
81675 München (DE)**

(54) **METHOD FOR PRODUCING RECYCLED WATER**

(57)     An object of the present invention is to provide a method for producing recycled water, according to which when recycled water is prepared from waste water, chemical cleaning of a membrane can be significantly reduced and fouling of the membrane can be eliminated by substantially only washing with water. The above object is achieved by providing a method for producing recycled water, the method including the following steps (A) to (D) of: (A) subjecting waste water to an anaerobic treatment and an aerobic treatment which are carried out by microorganisms, the waste water having been discharged during a production process of a PHA; (B) subjecting treated water obtained in step (A) to pretreatment filtration performed by a membrane bioreactor method; (C) subjecting the treated water obtained in step (B) to an alkali treatment; and (D) filtering, through an ion removal membrane, the treated water obtained in step (C).

EP 4 321 487 A1

**Description**

Technical Field

The present invention relates to a method for producing recycled water and a method for producing a polyhydroxyalkanoate (hereinafter, also referred to as "PHA").

Background Art

Biodegradable plastic is to be completely biodegraded by microorganisms etc. in soil or water, and is then incorporated into a natural carbon cycle process. Therefore, biodegradable plastic is desired to be used actively as an environmentally friendly plastic material that has little adverse impact on ecosystems. As ¥typical biodegradable plastic, biodegradable plastics, such as PHAs, derived from plants have attracted attention. A PHA is an aliphatic polyester (thermoplastic polyester), which is produced by microorganisms with use of a plant-derived natural organic acid or fat as a carbon source and is accumulated as an energy-accumulating substance in cells.

A PHA produced by microorganisms is water-insoluble and usually accumulates in microorganism cells as granules. Therefore, in order to utilize the PHA as a plastic, it is necessary to separate and take out the PHA from the microorganism cells. For example, Patent Literature 1 reports, as a method for separating and purifying a PHA, a method in which alkali addition and high-pressure crushing are combined. In addition, Patent Literature 2 reports, as a method for separating and purifying a PHA, a method in which physical cell crushing and a chemical treatment with use of an enzyme and a surfactant are combined.

However, in conventional methods for producing a PHA, an amount of waste water, such as waste water resulting from bacteria crushing and waste water resulting from centrifugation, tends to be large. Therefore, recycle of waste water has been desired in order to reduce an environmental load.

Citation List

[Patent Literature]

[Patent Literature 1]
Japanese Patent Application Publication Tokukaihei No. 07-31489
[Patent Literature 2]
Japanese Patent Application Publication Tokukai No. 2008-193940

Summary of Invention

Technical Problem

In a method for separating and purifying a PHA, it is common to use a filtration membrane in order to separate the PHA. Repeated use of the filtration membrane requires a membrane performance recovery treatment by chemical cleaning. However, in conventional technologies for waste water recycling, there has been room for improvement because there has been problems such as early deterioration of the above filtration membrane due to the membrane performance recovery treatment by chemical cleaning.

In light of the above, an object of an aspect of the present invention is to provide a method for producing recycled water, according to which when recycled water is prepared from waste water, chemical cleaning of a membrane can be significantly reduced and fouling of the membrane can be eliminated by substantially only washing with water.

Solution to Problem

In order to solve the above problem, the inventors of the present application made diligent studies. As a result, the inventors obtained novel findings. According to the findings, in a case where an alkali treatment step is performed before a membrane filtration step in a method for producing recycled water in which the recycled water is produced from waste water, fouling of a membrane can be eliminated by substantially only washing with water. As a result, the inventors have achieved the present invention.

Therefore, an aspect of the present invention is a method for producing recycled water, the method including the following steps (A) to (D) of: (A) subjecting waste water to an anaerobic treatment and an aerobic treatment which are carried out by microorganisms, the waste water having been discharged during a production process of a PHA; (B) subjecting treated water obtained in step (A) to pretreatment filtration performed by a membrane bioreactor method; (C) subjecting the treated water obtained in step (B) to an alkali treatment; and (D) filtering, through an ion removal membrane, the treated water obtained in step (C).

Advantageous Effects of Invention

An aspect of the present invention makes it possible to provide a method for producing recycled water, according to which when recycled water is prepared from waste water, fouling of a membrane can be eliminated by substantially only washing with water without chemical cleaning of the membrane.

Description of Embodiments

The following description will discuss an embodiment of the present invention in detail. Note that in the present specification, the wording "A to B" indicative of a numerical range means "A or more and B or less" unless otherwise specifically mentioned. In addition, all of the literatures listed herein are incorporated by reference herein.

[1. Outline of embodiment of present invention]

A method for producing recycled water (hereinafter, also referred to as the present production method) in accordance with an embodiment of the present invention includes the following steps (A) to (D): (A) a treatment step of subjecting waste water to an anaerobic treatment and an aerobic treatment which are carried out by microorganisms, the waste water having been discharged during a production process of a PHA; (B) a pretreatment filtration step of subjecting treated water obtained in step (A) to pretreatment filtration performed by a membrane bioreactor method (membrane separation activated sludge method); (C) an alkali treatment step of subjecting the treated water obtained in step (B) to an alkali treatment; and (D) a filtration step of filtering, through an ion removal membrane, the treated water obtained in step (C)

Membrane filtration method is a typical waste water recycling method. However, the membrane filtration method may result in fouling (clogging) due to a very small and highly sticky matter suspended in waste water. Therefore, washing of a membrane needs to be repeated for carrying out an operation. In general, the following two cleaning methods for membranes are known: (1) physical cleaning whose typical example is back pressure washing in which filtered water is caused to flow back from a permeation side (secondary side); and (2) chemical cleaning with use of a chemical.

In the above physical cleaning, a reversible substance among substances adhered to a surface or inside of a membrane can be removed by automatically carrying out washing with use of water at regular intervals (approximately once every 30 minutes to 1 hour). However, the physical cleaning cannot completely remove fouling substances adhered to membrane pores, etc., so that a transmembrane pressure difference continues to increase. On this account, when the transmembrane pressure difference increased to some extent, it was necessary to carry out chemical cleaning in order to remove fouling substances from the membrane. However, there was a problem in that when cleaning with a chemical was repeated, the action of the chemical damaged the membrane and further made the membrane unable to be used as ion removal membrane. That is, there has been a demand for a method for extending a usable period of the membrane.

In light of the above, in order to extend a usable period of a membrane, the present inventors made diligent studies to provide a method for producing recycled water without need for chemical cleaning, and found the following.

- Particular metal ions cause irreversible fouling of a membrane.
- The irreversible fouling on the ion removal membrane can be suppressed, in a case where treated water is subjected to an alkali treatment step prior to carrying out the filtration step with use of an ion removal membrane and a specific fouling substance contained in the treated water is precipitated as a solid in advance.
- Fouling of the membrane can be eliminated by only washing with water, in a case where the treated water is subjected to the alkali treatment step prior to carrying out the filtration step with use of the ion removal membrane.

A method for producing recycled water that includes a characteristic step as described above has not conventionally existed, but is an excellent technique.

In addition, surprisingly, the inventors succeeded in obtaining a PHA that is highly thermostable when the inventors produced the PHA with use of recycled water that had been obtained by the aforesaid production method. The following will describe embodiments of the present invention in detail.

Note that, in the present specification, the wording "substantially only washing with water" is intended to mean that (i) it is only necessary that washing with water is a main cleaning aspect and (ii) another cleaning aspect can be included in addition to washing with water. For example, "substantially only washing with water" may include, in addition to washing with water, chemical cleaning in which the amount of a chemical used is lower than usual. The wording "substantially only washing with water" intended to mean preferably only cleaning with water.

[2. Method for producing recycled water]

The present production method includes the following steps (A) to (D) of:

- Step (A): a treatment step of subjecting waste water to an anaerobic treatment and an aerobic treatment which are carried out by microorganisms, the waste water having been discharged during a production process of a PHA;
- Step (B): a pre-treatment filtration step of subjecting treated water obtained in step (A) to pretreatment filtration performed by a membrane bioreactor method;
- Step (C): an alkali treatment step of subjecting the treated water obtained in step (B) to an alkali treatment; and - Step (D): a filtration step of filtering, through an ion removal membrane, the treated water obtained in step (C).

(Step (A))

Step (A) in the present production method is a treatment step of subjecting waste water to an anaerobic treatment and an aerobic treatment which are carried out by microorganisms, the waste water having been discharged during a production process of a PHA. With step (A), an organic substance contained in the waste water can be degraded.
The anaerobic treatment and the aerobic treatment which are carried out by microorganisms are not particularly limited, and can be carried out by a general method that is used in water treatment. The anaerobic treatment may be carried out, for example, by: degrading high molecular weight carbohydrates, lipids, etc. to an organic acid, a lower alcohol, etc., by the action of acidogenic bacteria; and thereafter, degrading the organic acid, the lower alcohol, etc. to methane gas and carbon dioxide, by the action of granular methanogens. The anaerobic treatment device can include, for example, an acidogenesis tank in which a reaction is carried out by the acidogenic bacteria and an EGSB-type methanogenisis tank in which a reaction is carried out by the methanogens. The aerobic treatment can be carried out, for example, by: degrading, by the action of aerobic bacteria in an aeration tank, an organic substance that has not been degraded in the anaerobic treatment. In this case, used are a device including a denitrification tank (activated sludge treatment tank) and the aeration tank (activated sludge treatment tank). An aerobic treatment device may include, for example, a denitrification tank (activated sludge treatment tank), an aeration tank (activated sludge treatment tank), a second denitrification tank (activated sludge treatment tank), and a re-aeration tank (activated sludge treatment tank).

(Step (B))

Step (B) in the present production method is a pretreatment filtration step of subjecting treated water obtained in step (A) to pretreatment filtration performed by a membrane bioreactor method. With step (A), a substance which has a large particle diameter in the waste water and which has not been degraded in step (A) can be degraded.
The pre-treatment filtration step carried out by the membrane bioreactor method is not particularly limited, and can be carried out by a general method that is used in water treatment. The pre-treatment filtration step can be carried out, for example, in a device obtained by providing a membrane bioreactor method permeation membrane, which is a UF membrane or an MF membrane, in the aeration tank (activated sludge treatment tank) or the re-aeration tank (activated sludge treatment tank).
The MF membrane may have membrane pores that can be, but are not particularly limited to, for example, approximately 0.4 $\mu$m pores. Meanwhile, the UF membrane may have membrane pores that can be, but are not particularly limited to, for example, approximately 0.05 um pores.

(Step (C))

Step (C) in the present production method is an alkali treatment step of subjecting the treated water obtained in step (B) to an alkali treatment. With step (C), a substance (i.e., a fouling substance) that fouls the ion removal membrane in step (D) described below can be precipitated in advance. This makes it possible to remove, by only washing with water in step (D), the fouling substance that has adhered to the ion removal membrane.
More specifically, in the absence of the alkali treatment of step (C), the salt concentration of water that does not permeate the membrane becomes higher in the filtration step with use of a membrane in step (D). As a result, crystals are precipitated on a surface of the ion removal membrane. Then, if filtration is further continued, the crystals will grow more due to repeated precipitation of the crystals. As a result, the crystals adhere tightly to the ion removal membrane and occlude the membrane. Therefore, in conventional techniques, it has been necessary to remove, by carrying out chemical cleaning, such crystals adhered to the surface of the membrane. However, in a case where the alkali treatment is carried out in step (C), the crystals are precipitated before filtration with use of the membrane, so that the crystals do not adhere tightly to the membrane.

In other words, according to the present production method, the membrane can be recovered by substantially only washing with water, without chemical cleaning of the ion removal membrane. As a result, the ion removal membrane is not damaged by a chemical and no irreversible fouling of the ion removal membrane occurs. This makes it possible to use the ion removal membrane for a long period of time. In addition, since the chemical normally used for cleaning the ion removal membrane is a dangerous drug, it is preferable to avoid the use of such a chemical also in terms of environmental protection.

In the present specification, the term "alkali treatment" is intended to mean adjustment of parameters such as pH, fouling index (FI) value, turbidity, etc. of the treated water by, for example, adding an alkali substance to the treated water.

The method for carrying out the alkali treatment of step (C) is not particularly limited, provided that the method allows for adjustment of each of the parameters of the treated water to a desired value. In view of easy adjustment of each of the parameters, it is preferable to carry out the alkali treatment by adding an alkali aqueous solution.

Examples of the alkali aqueous solution used in step (C) include, but are not limited to, the following: aqueous solutions of alkali metal hydroxides, such as sodium hydroxide, potassium hydroxide, and lithium hydroxide; aqueous solutions of alkali metal carbonates, such as sodium carbonate, and potassium carbonate; aqueous solutions of alkali metal hydrogen carbonates such as sodium hydrogen carbonate and potassium hydrogen carbonate; aqueous solutions of organic acid alkali metal salts, such as sodium acetate and potassium acetate; aqueous solutions of alkali metal borates, such as borax; solutions of alkali metal phosphates, such as trisodium phosphate, disodium hydrogen phosphate, tripotassium phosphate, and dipotassium hydrogen phosphate; aqueous solutions of alkali earth metal hydroxides, such as barium hydroxide; and ammonia water. From the viewpoint of cost reduction, it is preferable to use an aqueous solution of sodium hydroxide.

In step (C), it is preferable to adjust the pH of the treated water to 7.0 to 11.0, more preferably to 8.0 to 11.0, and even more preferably to 8.5 to 11.0. If the pH is not less than 7.0, a metal salt is precipitated in the treated water. In addition, if the pH is not more than 11.0, deterioration of the ion removal membrane can be prevented.

In step (C), the turbidity of the treated water is preferably not less than 0.1, more preferably not less than 0.2, and even more preferably not less than 0.3. If the turbidity is not less than 0.1, a solid content has been sufficiently precipitated. Further, the upper limit of the turbidity of the treated water is preferably not more than 30, more preferably not more than 20, and even more preferably not more than 10, and particularly preferably not more than 5. If the turbidity is not more than 30, filtration of the treated water is not hindered. Note that the turbidity of the treated water is measured by a method described in Examples later.

In step (C), the FI value of the treated water is preferably not less than 4.5, more preferably not less than 5.0, even more preferably not less than 5.5, still more preferably not less than 6.0, and particularly preferably not less than 6.5. If the FI value is in the range described above, then the solid content has been sufficiently precipitated in the treated water. The upper limit of the FI value is not particularly limited. The "FI value" here is derived by the formula (1) below.

$$FI \, value = \frac{PF}{T} = \frac{100(1 - T1/T2)}{T} \quad \cdots \, (1)$$

In the formula (1), PF represents an occlusion factor; T1 represents the time required for 500 mL of the treated water to permeate the ion removal membrane; and T2 represents the time required for another 500 mL of the treated water to permeate the same ion removal membrane after measuring T1. Further, T represents the time from the time when measurement of T1 starts to the time when measurement of T2 starts.

Conventionally, it has been necessary to carry out a pre-treatment so that the FI value of the treated water used for permeation of the ion removal membrane (e.g., an RO membrane) can be 0 to 4. Thus, it is surprising that in the present production method, an advantageous effect of the present invention can be obtained with use of the treated water having a high FI value (e.g., not less than 4.5) since the treated water having such a high FI value is not normally allowed to permeate the ion removal membrane.

The present production method preferably further includes the following step (C') in step (C):

- Step (C'): a precipitation step of precipitating a solid containing a polyvalent ion in the treated water.

The polyvalent ion is not normally precipitated in the treated water, and may be a cause of fouling of the ion removal membrane. On this account, step (C'), in which a solid containing the polyvalent ion is precipitated in advance, is carried before the treated water is caused to permeate the ion removal membrane. This makes it possible to further suppress fouling of the ion removal membrane.

A method for precipitating the solid is not particularly limited, and can be carried out by, for example, pH adjustment, condensation, temperature adjustment, and/or the like.

Examples of the polyvalent ion include, but are not limited to, divalent or higher valence cations and divalent or higher valence anions. The number of types of polyvalent ions contained in the solid is not particularly limited, and may be only one or two or more. Further, the solid that precipitates may be in the form of crystals, particles, rods, or the like.

In an embodiment of the present invention, it is preferable that the polyvalent ion be at least one selected from the group consisting of $Si^{2+}$, $Ca^{2+}$, $PO_4^{2-}$, $SO_4^{2-}$, $Mg^{2+}$, $Mn^{2+}$, $Zn^{2+}$, $Fe^{2+}$, $Fe^{3+}$, $Sr^{2+}$, $Cu^{2+}$, $Al^{3+}$, and $Sn^{3+}$.

Generally, monovalent ions such as $Na^+$, $K^+$, and $NH^{4+}$, and anions such as $Cl^-$, and $NO^{3-}$ are unlikely to be responsible for fouling because these ions are highly soluble in water. On the other hand, the above-described polyvalent ions may be a cause of fouling of the ion removal membrane because these polyvalent ions are less soluble in water and easy to precipitate as a solid.

The present production method preferably further includes the following step (C") in step (C):

- Step (C"): a sedimentation removal step of removing, by sedimentation, the solid that has precipitated in the treated water in step (C')
  Including step (C"), the present production method makes it possible to further reduce, in step (D) described below, the frequency of cleaning of the ion removal membrane.

A method for carrying out the sedimentation removal of the solid that has been precipitated is not particularly limited. The sedimentation removal of the solid can be carried out, for example, by a centrifugal settler or a thickener.

In an embodiment of the present invention, a scale inhibitor may be added when step (C) is carried out. The addition of the scale inhibitor makes further growth of the crystals on the surface of the ion removal membrane difficult. Examples of the scale inhibitor that can be used include, but are not limited to, for example, Genesys LF (manufactured by Genesys), PC-191T (manufactured by Katayama Nalco Inc.), and Kuriverter N series (Kurita Water Industries Ltd.). Further, the amount of the scale inhibitor added is also not particularly limited, and may be, for example, 1 ppm to 50 ppm.

(Step (D))

Step (D) in the present production method is a filtration step of filtering, through the ion removal membrane, the treated water obtained in step (C). Step (D) makes it possible to remove the solid that has precipitated in step (C). The treated water having undergone step (D) becomes recycled water.

In the present specification, the term "recycled water" is intended to mean water that is usable for production of a PHA, the water being obtained by carrying out the steps (A) to (D) on waste water which has been discharged during a production process of a PHA.

When a pressure of 3000 kPa at 20°C is applied, the ion removal membrane in step (D) has a $MgSO_4$ blocking rate of preferably 60% to 100%, more preferably 70% to 100%, and even more preferably 90% to 100%. In a case where the $MgSO_4$ blocking rate is not less than 60%, the ion removal membrane does not have an increased ion permeability. In addition, in a case where resultant recycled water is used for purification of the PHA, a decrease in molecular weight at a high temperature is likely to be suppressed.

The transmembrane pressure difference of the ion removal membrane in step (D) is not particularly limited, but in terms of the ion removal rate, the transmembrane pressure difference is preferably 0.4 MPa to 4.14 MPa, more preferably 0.5 MPa to 2.5 MPa, and even more preferably 0.6 MPa to 2.0 MPa. In a case where the transmembrane pressure difference is not less than 0.4 MPa, the amount of permeable water and the ion removal rate do not decrease. Meanwhile, in a case where the transmembrane pressure difference is not more than 4.14 MPa, the membrane is unlikely to break. Note that the transmembrane pressure difference of the ion removal membrane is measured by a method described in Examples described later.

The timing of carrying out cleaning of the ion removal membrane is not particularly limited. It is preferable to carry out the cleaning at the time when the transmembrane pressure difference of the ion removal membrane becomes preferably not less than 4.14 MPa, more preferably not less than 2.5 MPa, and even more preferably not less than 2.0 MPa. In a case where the cleaning of the ion removal membrane is carried out at the time when the transmembrane pressure difference of the ion removal membrane becomes not less than 4.14 MPa, the transmembrane pressure difference is maintained in the above preferable range. A time for which the cleaning of the ion removal membrane is carried out is not particularly limited, provided that the transmembrane pressure difference of the ion removal membrane can be sufficiently reduced. Such a time is, for example, not less than 30 minutes.

The ion removal membrane in step (D) has a permeation rate of preferably 0.01 L/min to 2000 L/min, and more preferably 0.5 L/min to 1500 L/min. A permeation rate of not less than 0.01 L/min increases productivity. In addition, if the permeation rate is not more than 2000 L/min, the ion removal membrane is less likely to break.

The treated water during filtration in step (D) is at a temperature that is not particularly limited but is preferably not higher than 50°C, and more preferably not higher than 45°C. When the water temperature of the treated water is not more than 50°C, the membrane is not likely to deteriorate. Although the lower limit of the water temperature of the treated water is not particularly limited, the lower limit is preferably not lower than 1°C from the viewpoint of smooth filtration.

In step (D), in order to remove the metal salt precipitated, it is preferable to clean the ion removal membrane at regular intervals.

As a method for cleaning the ion removal membrane, it is preferable to use cleaning by flushing. Cleaning by flushing is physical cleaning in which in order to remove initial contamination on the ion removal membrane, a high flow rate of water is caused to flow at a low pressure. Water used for the cleaning by flushing may be simply tap water, but in view of efficiency and cost, it is preferable to use the recycled water that has permeated through the ion removal membrane. The pressure at the time of cleaning by flushing may be set such that the primary pressure of the ion removal membrane is not more than 0.29 MPa. Further, the flow rate of the recycled water may be set to not less than 6 m/min.

As the ion removal membrane, it is preferable to use, because of high ion (e.g., calcium ion) removal performance, one or more selected from the group consisting of NF membranes and RO membranes, and it is more preferable to use an RO membrane.

[3. Method for producing PHA]

A PHA production method in accordance with an embodiment of the present invention is a method for producing a PHA (hereinafter, also referred to as "the present PHA production method"), including the steps of: (a) crushing or solubilizing microorganism cells that contain a PHA; and (b) separating the PHA in a composition obtained in step (a), the steps (a) and (b) using recycled water produced by the present production method. In the present PHA production method, use of the recycled water produced by the present production method can improve thermal stability of the PHA.

(Microorganism cells containing PHA)

In an embodiment of the present invention, microorganism cells containing a PHA can be obtained by culturing a microorganism that has a PHA production capacity.

In an embodiment of the present invention, the PHA is a generic term for polymers each of which contains a 3-hydroxybutyrate (hereafter, also referred to as "3HB") as a monomer unit. The PHA may be poly(3-hydroxybutyrate) which results from homopolymerization using 3-hydroxybutyrate as a monomer unit, or a copolymer using 3-hydroxybutyrate and another 3-hydroxyalkanoic acid as monomers. Examples of such other 3-hydroxyalkanoic acid include 3-hydroxyhexanoic acid (hereinafter, also referred to as "3HH"), 3-hydroxyheptanoic acid, 3-hydroxyoctanoic acid, 3-hydroxynonanoic acid, 3-hydroxydecanoic acid, 3-hydroxyundecanoic acid, 3-hydroxydodecanoic acid, 3-hydroxytridecanoic acid, 3-hydroxytetoradecanoic acid, 3-hydroxypentadecanoic acid, and 3-hydroxyhexadecanoic acid.

In terms of easy industrial production, the PHA is preferably poly(3-hydroxybutyrate), poly(3-hydroxybutyrate-co-3-hydroxyhexanoate), poly(3-hydroxybutyrate-co-3--3-hydroxyoctanoate), or the like, and particularly preferably poly (3-hydroxybutyrate-co-3-hydroxyhexanoate). The composition ratio of each monomer unit constituting a two-component copolymer PHBH of the above-mentioned 3HB and 3HH is not particularly limited, but when the total of all monomer units is assumed to be 100 mol%, the composition ratio of a 3HH unit may be 1 mol% to 50 mol%, 1 mol% to 25 mol%, or 1% to 15 mol%.

In an embodiment of the present invention, the microorganism having the PHA production capacity is not particularly limited. It is possible to use: a microorganism isolated from nature; a microorganism deposited at a depositary institution (for example, IFO, ATCC, or the like) for strains; or a mutant, a transformant, or the like that can be prepared from any of the aforesaid microorganisms. Examples of the microorganism include bacteria of the genera Cupriavidus, Alcaligenes, Ralstonia, Pseudomonas, Bacillus, Azotobacter, Nocardia, and Aeromonas. In particular, the microorganism is preferably a strain of Alcaligenes lipolytica (A. lipolytica), Alcaligenes latus (A. latus), Aeromonas caviae (A. caviae), Aeromonas hydrophila (A. hydrophila), Capriavidus necator (C. necator), or the like. In a case where the microorganism is inherently not capable of producing a PHA or in a case where the microorganism produces only a small amount of a PHA, a transformant obtained by introducing, into the microorganism, an intended-PHA synthase gene and/or a variant of the gene can be also used. The PHA synthase gene to be used for preparation of the transformant is not particularly limited, but is preferably a PHA synthase gene derived from Aeromonas caviae.

By culturing the above-described microorganism under appropriate conditions, it is possible to obtain microorganisms having a PHA accumulated within cells of the microorganisms. A method of culturing the microorganism is not particularly limited. As the method, for example, a method described in, for example, Japanese Patent Application Publication Tokukaihei No. 05-93049 and International Publication No. WO 2008/010296, can be employed. As the microorganism cells containing the PHA, a bacteria culture fluid containing PHA-containing microorganism cells after completion of culture can be used as is. Alternatively, as the microorganism cells containing the PHA, a sterilized bacteria culture fluid can be used after the bacteria have been killed by heating the bacteria culture fluid. Such sterilization may be carried out, for example, by heat treatment at a temperature of 50°C to 80°C for 5 minutes to 120 minutes.

(Step (a))

In step (a), microorganism cells that contain a PHA is crushed or solubilized. Step (a) can be carried out with use of at least one treatment selected from the group consisting of, for example, chemical treatments and physical crushing treatments.

Such a chemical treatment can be carried out with use of at least one compound selected from the group consisting of an alkali compound, a proteolytic enzyme and a cell wall degrading enzyme.

The alkali compound is not particularly limited, provided that cell walls of the PHA-containing microorganism cells can be crushed and the PHA in those cells can be caused to flow out of the cells. Examples of the alkali compound include: alkali metal hydroxides, such as sodium hydroxide, potassium hydroxide, and lithium hydroxide; alkali metal carbonates, such as sodium carbonate, and potassium carbonate; alkali metal hydrogen carbonates such as sodium hydrogen carbonate and potassium hydrogen carbonate; organic acid alkali metal salts, such as sodium acetate and potassium acetate; alkali metal borates, such as borax; alkali metal phosphates, such as trisodium phosphate, disodium hydrogen phosphate, tripotassium phosphate, and dipotassium hydrogen phosphate; alkali earth metal hydroxides, such as barium hydroxide; and ammonia water. Among others, sodium hydroxide, sodium carbonate, potassium hydroxide, and lithium hydroxide are preferable from the viewpoint of suitability to industrial production and cost reduction.

Examples of the proteolytic enzyme include, but are not limited to, alcalase, pepsin, trypsin, papain, chymotrypsin, aminopeptidase, and carboxypeptidase. Specific examples of the proteolytic enzyme include "Protease A," "Protease P" and "Protease N" (which are manufactured by Amano Enzyme Inc.), and "Alcalase", "Esperase", "Sabinase", and "Evalase" (which are manufactured by Novozymes A/S). These enzymes can be used industrially and can also be suitably used in terms of degradation activity.

Examples of the cell wall degrading enzyme include, but are not limited to, lysozyme, amylase, cellulase, maltase, saccharase, $\alpha$-glycosinase, and $\beta$-glycosinase. Among the aforesaid cell wall degrading enzymes, it is preferable to use lysozyme in terms of a lytic effect. Specific examples of the cell wall degrading enzyme include "Lysozyme" (available from Shangdong Huayuan Economic & Trade Co., Ltd.), "Biozyme A", "Cellulase A 'Amano' 3", "Cellulase T 'Amano' 4", and "a-glucosidase 'Amano'" (which are manufactured by Amano Enzyme Inc.), and "Termamyl" and "Cellusoft" (which are manufactured by Novozymes A/S).

Treatment with any of the above-described enzymes is desirably carried out in the presence of a surfactant because a high separating and purifying effect can be obtained. Further, it is possible to use, as the enzyme, for example, an enzyme composition that contains an enzyme and at least one additive selected from the group consisting of an enzyme stabilizer, a surfactant, and a re-contamination inhibitor.

The surfactant includes an anionic surfactant, a cationic surfactant, an amphoteric surfactant, and a nonionic surfactant. In terms of high removal efficacy of a residue resulting from crushing of cell membranes, the surfactant is preferably an anionic surfactant and/or a nonionic surfactant. For the purpose of removing a protein etc., it is preferable to use the anionic surfactant. Meanwhile, for the purpose of removing a fatty acid and/or a fat, it is preferable to use the nonionic surfactant. Further, it is possible to use both of the anionic surfactant and the nonionic surfactant. In a case where both of such surfactants are used, the weight ratio of the anionic surfactant/nonionic surfactant is preferably 1/100 to 100/10, more preferably 5/100 to 100/20, even more preferably 5/100 to 100/100, and particularly preferably 5/100 to 50/100.

Examples of the anionic surfactant include alkyl sulfates, alkylbenzene sulfonates, alkyl sulfate ester salts, alkenyl sulfate ester salts, alkyl ether sulfate ester salts, alkenyl ether sulfate ester salts, $\alpha$-olefin sulfonates, $\alpha$-sulfofatty acid salts, esters of $\alpha$-sulfofatty acid salts, alkyl ether carboxylates, alkenyl ether carboxylates, amino acid type surfactants, and N-acylamino acid type surfactants. Among others, an alkyl sulfate which has an alkyl group having 12 to 14 carbon atoms, a linear alkyl benzene sulfonate which has an alkyl group having 12 to 16 carbon atoms, and an alkyl sulfate ester salt or alkyl ether sulfate ester salt which has an alkyl group having 10 to 18 carbon atoms are preferable. Meanwhile, preferable examples of a counter ion include alkali metals such as sodium and potassium, alkali earth metals such as magnesium, and alkanolamines such as monoethanolamines, diethanolamines, and triethanolamines.

Examples of the nonionic surfactant include polyoxyethylene alkyl ether, polyoxyethylene alkyl phenyl ether, polyoxyalkylene alkyl ether, sorbitan fatty acid ester, alkyl polyglucoside, fatty acid diethanolamide, and alkyl monoglyceryl ether. In view of high hydrophilicity and relatively good biodegradability, polyoxyethylene alkyl ether or polyoxyalkylene alkyl ether is preferable.

Examples of the cationic surfactant include alkyl trimethyl ammonium salts and dialkyl dimethyl ammonium salts.

The amphoteric surfactant include carbobetaine type and the sulfobetaine type.

Among the surfactants mentioned above, sodium dodecyl sulfate, sodium dodecyl benzene sulfonate, sodium cholate, sodium deoxycholate and sodium oleate, which are anionic surfactants, and polyoxyethylene alkyl ether and polyoxyalkylene alkyl ether, which are nonionic surfactants, and the like are preferable in terms of cost, amount used and an effect of addition.

The amount of the surfactant added is not particularly limited, but is preferably 0.001 parts by weight to 10 parts by weight and further in view of cost, preferably 0.001 parts by weight to 5 parts by weight, relative to 100 parts by weight of the bacteria culture fluid. It is possible to use one type of the aforesaid surfactants alone or to use two or more types of the surfactants in combination.

It is preferable that an enzyme treatment be carried out, for example, while stirring is carried out after addition of the alkali compound and/or the surfactant to the bacteria culture fluid. As to enzymatic treatment conditions, the enzyme treatment is carried out preferably under control of an optimal value of the enzyme used. The amount of the enzyme required depends on the type and activity of the enzyme. The amount of the enzyme required is not particularly limited, but is preferably 0.001 parts by weight to 10 parts by weight and further in view of cost, preferably 0.001 parts by weight to 5 parts by weight, relative to 100 parts by weight of the PHA.

The physical crushing treatment is preferably carried out after addition of an alkali compound or addition of an alkali compound and a surfactant, in terms of enhancing a crushing effect and of easy collection of the PHA. As the alkali compound and the surfactant, any of the above-described alkali compounds and any of the above-described surfactants can be used as appropriate. Among the above-described alkali compounds, in terms of suitability to industrial production and cost reduction, sodium hydroxide, sodium carbonate, potassium hydroxide, and lithium hydroxide are preferable as the alkali compound. Among the above-described surfactants, sodium dodecyl sulfate, sodium dodecyl benzene sulfonate, sodium cholate, sodium deoxycholate and sodium oleate, which are anionic surfactants, and polyoxyethylene alkyl ether and polyoxyalkylene alkyl ether, which are nonionic surfactants, and the like are preferable in terms of cost, amount used and an effect of addition.

It is preferable to adjust the pH of the bacteria culture fluid to 8.0 to 12.5 by adding the alkali compound. It is easy to solubilize residues of bacteria (microorganism cells), organic substances produced by bacteria, constituent organic substances of bacteria, etc. without affecting the PHA. After adding the alkali compound to the bacteria culture fluid, the physical crushing treatment may be carried out for 30 minutes to 2 hours at a temperature of 20°C to 80°C and preferably at a temperature of 20°C to 50°C.

The amount of the surfactant added is not particularly limited, but is preferably 0.001 parts by weight to 10 parts by weight and further in view of cost, preferably 0.001 parts by weight to 5 parts by weight, relative to 100 parts by weight of the bacteria culture fluid. It is possible to use one type of the aforesaid surfactants alone or to use two or more types of the surfactants in combination.

Examples of an apparatus for carrying out the physical crushing treatment include, but are not limited to, a high-pressure homogenizer, an ultrasonic crusher, an emulsifying disperser, and a bead mill. Among others, the use of a high pressure homogenizer is preferable in terms of crushing efficiency. More preferable is a type in which a suspension is introduced into a pressure-resistant container having a minute opening and extruded from the opening by application of high pressure. Examples of the crusher of this type include a high pressure homogenizer model "PA2K type" manufactured by Niro Soavi S.p.A. The use of the high pressure homogenizer exerts a large shearing force on the microorganism cells. This efficiently crushes the microorganism cells and consequently improves separability of the PHA. Because such a device is subject to high pressure at the opening and become hot instantaneously, it is preferable to carry out the crushing treatment at 20°C to 40°C by cooling the bacteria culture fluid with use of a general cold constant-temperature circulatory bath and preventing an increase in temperature, when necessary. Carrying out such a treatment at 20°C to 40°C makes it possible to carry out a treatment while hardly reducing the molecular weight of the PHA. A crushing pressure at the time when the high-pressure crushing is carried out is not particularly limited but is preferably 30 MPa to 60 MPa in terms of crushing efficiency and cost.

In step (a), the chemical treatment and the physical crushing treatment may be used in combination. In this case, it is preferable to carry out the chemical treatment followed by the physical crushing treatment, in order to enhance the crushing effect. In terms of cost, step (a) may be carried out by only the physical crushing treatment.

In step (a), the recycled water that has been produced by the present production method can be used as water. The recycled water may be used, for example, when the surfactant, the alkali compound, and the like are added. With use of the recycled water, it becomes possible to reduce water consumption. This leads to reduction in cost. In addition, the use of the recycled water improves thermal stability of a resultant PHA.

In the recycled water, the concentration of calcium ions is preferably not more than 4.5 mg/L, more preferably not more than 3.0 mg/L, and even more preferably not more than 2.0 mg/L. In a case where the concentration of the calcium ions in the recycled water is not more than 4.5 mg/L, the PHA pyrolysis reducing effect is brought about. Further, in the recycled water, the concentration of sodium ions is preferably not more than 450 mg/L, more preferably not more than 250 mg/L, and even more preferably not more than 220 mg/L. In a case where the concentration of the sodium ions in the recycled water is not more than 450 mg/L, the PHA pyrolysis reducing effect is brought about.

(Step (b))

In step (b), the composition obtained in step (a), for example, the PHA in a crushed solution is separated. The separation is not particularly limited, and solid-liquid separation can be carried out with use of a method such as filtration, sedimentation, centrifugation, etc., and the PHA can be collected as a water-insoluble component. Centrifugation is preferable since centrifugation allows for industrial mass treatment and also allows for continuous operation.

A centrifuge is not particularly limited. The centrifuge is preferably a centrifugal settler having a poreless rotary container. Examples of such a type of the centrifuge include a disk stack type, a cylinder type, and a decanter type. Because the difference between a specific gravity of PHA particles and the specific gravity of water is small, it is preferable to use the disk stack type (intermittent discharge type, or nozzle discharge type) which has a large sedimentation separation area and which can provide a high acceleration rate. In a case where the concentration of the PHA contained in the crushing treatment solution is high, the centrifuge of the nozzle discharge type is particularly preferable. As the decanter type, a model having a separation plate and a large sedimentation separation area is preferable.

In step (b), prior to separation, with respect to the composition obtained in step (a), for example, to 100 parts by weight of the crushed solution, 500 parts by weight to 1000 parts by weight of an aqueous medium may be added.

In step (b), the aqueous medium may be the recycled water produced by the present production method or a mixture solvent of the recycled water and a water-miscible organic solvent. The content of the recycled water in the aqueous medium is preferably not less than 50% by weight, more preferably not less than 70% by weight, and even more preferably not less than 80% by weight, and particularly not less than 85% by weight.

In step (b), in the recycled water, the concentration of calcium ions is preferably not more than 4.5 mg/L, more preferably not more than 3.0 mg/L, and even more preferably not more than 2.0 mg/L. In a case where the concentration of the calcium ions in the recycled water is not more than 4.5 mg/L, the PHA pyrolysis-reducing effect is brought about. In the recycled water, the concentration of sodium ions is preferably not more than 450 mg/L, more preferably not more than 250 mg/L, and even more preferably not more than 220 mg/L. In a case where the concentration of the sodium ions in the recycled water is not more than 450 mg/L, the PHA pyrolysis-reducing effect is brought about.

Examples of the water-miscible organic solvent include, but are not particularly limited to, the following: alcohols such as methanol, ethanol, 1-propanol, 2-propanol, 1-butanol, 2-butanol, iso-butanol, pentanol, hexanol, and heptanol; ketones such as acetone, and methyl ethyl ketone; ethers such as tetrahydrofuran, and dioxane; nitriles such as acetonitrile and propionitrile; amides such as dimethylformamide and acetamide; and dimethyl sulfoxide, pyridine, and piperidine. Among others, for example, the following are preferable in view of easy removal: methanol, ethanol, 1-propanol, 2-propanol, 1-butanol, 2-butanol, iso-butanol, acetone, methyl ethyl ketone, tetrahydrofuran, dioxane, acetonitrile, and propionitrile. Further, for example, the following are preferable in view of easy availability: methanol, ethanol, 1-propanol, 2-propanol, butanol, and acetone. In addition, methanol, ethanol, and acetone are particularly preferable.

In step (b), the PHA may be purified by washing the PHA (water-insoluble component containing PHA), which has been separated, at least one time with use of the aqueous medium described above. The aqueous medium may be added in an amount of, for example, 500 parts by weight to 1000 parts by weight with respect to 100 parts by weight of the water-insoluble component containing the PHA. In addition, in order to effectively remove impurities derived from the microorganism cells and enhance a purification effect, an alkali compound, a surfactant, a proteolytic enzyme, etc. may be added to the aqueous medium.

(Step (c))

In an embodiment of the present invention, the present method for producing a PHA may include step (c) of drying the PHA separated in step (b). The PHA (dehydrated resin) that has been washed with an aqueous medium and dehydrated can be dried as it is. As a result, the PHA in powder form can be obtained. A drying method can be selected as appropriate, and is not particularly limited. As the drying method, it is possible to use preferably a general drying method, such as spray drying, airflow drying, flow drying, or band drying.

In an embodiment of the present invention, after a dispersing agent is added to a PHA dispersion that has been washed with an aqueous medium and concentrated and pH of the PHA dispersion is adjusted to not more than 7, the PHA in powder form can be obtained by drying. Examples of the dispersing agent include the following: water-soluble polymers such as polyvinyl alcohol (PVA), methyl cellulose, ethyl cellulose, hydroxymethyl cellulose, hydroxyethyl cellulose, polyacrylic acid, sodium polyacrylate, potassium polyacrylate, polymethacrylic acid, and sodium polymethacrylate; and nonionic surfactants such as a polyethylene glycol-polypropylene glycol-block ether type (polyoxyethylene-polyoxypropylene-block polymer type). Examples of a method of adjusting pH to not more than 7 include a method in which an acid is added. The acid is not particularly limited but can be an organic acid or an inorganic acid. For example, sulfuric acid, chloric acid, phosphoric acid, or acetic acid can be used as appropriate.

The molecular weight of the PHA is not particularly limited, provided that the PHA exhibits a substantially sufficient physical property for an intended application. For example, in terms of shaping processability and strength of a shaped product, the weight average molecular weight of the PHA is preferably 50,000 to 3,000,000, and more preferably 60,000 to 1,500,000. Note that the weight average molecular weight here is measured from a polystyrene equivalent molecular weight distribution with use of gel permeation chromatography (GPC) with use of a chloroform eluent. As a column in the GPC, a column appropriate for measuring the molecular weight may be used.

It is preferable that the PHA be highly thermostable. When heat-treated at 160°C for 20 minutes, a weight average molecular weight retention rate of the PHA be not less than 70%, more preferably not less than 73%, even more preferably not less than 75%, even more preferably not less than 78%, and particularly preferably not less than 80%. Note that the thermal stability of the PHA is determined by a method described in Example described below.

Preferably, the PHA has a good color tone. Further, it is preferable that a yellowness index (YI value) of a sheet of the PHA that has been press-molded at 160°C so as to have a thickness of 5 mm be not more than 20, and more preferably not more than 17.

The PHA can be shaped into any of a variety of shaped products, such as various fibers, threads, ropes, textiles, knitted goods, nonwoven fabrics, papers, films, sheets, tubes, plates, rods, containers, bags, parts, foamed products, and the like. Such shaped products can be suitably used in agriculture, fisheries, forestry, horticulture, medicine, sanitary items, clothing, non-clothing, packaging, and other fields.

In the case of other biodegradable plastic produced by microorganisms, it is possible to use the recycled water that is produced by the present production method in the steps of: crushing or solubilizing microorganism cells containing biodegradable plastic; and separating the biodegradable plastic.

The present invention is not limited to the embodiments, but can be altered by a skilled person in the art within the scope of the claims. The present invention also encompasses, in its technical scope, any embodiment derived by combining technical means disclosed in differing embodiments.

In other words, an embodiment of the present invention encompasses the following.

<1> A method for producing recycled water, the method including the following steps (A) to (D) of:

(A) subjecting waste water to an anaerobic treatment and an aerobic treatment which are carried out by microorganisms, the waste water having been discharged during a production process of a polyhydroxyalkanoate;
(B) subjecting treated water obtained in step (A) to pretreatment filtration performed by a membrane bioreactor method;
(C) subjecting the treated water obtained in step (B) to an alkali treatment; and
(D) filtering, through an ion removal membrane, the treated water obtained in step (C).

<2> The method according to <1>, wherein in step (C), pH of the treated water is adjusted to 7 to 11, turbidity of the treated water is adjusted to not less than 0.1, and FI value of the treated water is adjusted to not less than 4.5.

<3> The method according to <1> or <2>, wherein in step (C), FI value of the treated water is adjusted to not less than 6.0.

<4> The method according to any one of <1> to <3>, wherein the alkali treatment is carried out with use of at least one selected from the group consisting of an aqueous solution of an alkali metal hydroxide, an aqueous solution of an alkali metal carbonate, an aqueous solution of an alkali metal hydrogen carbonate, an aqueous solution of organic acid alkali metal, an aqueous solution of an alkali metal borate, an aqueous solution of an alkali metal phosphate, an aqueous solution of an alkali earth metal hydroxide, and ammonia water.

<5> The method according to any one of <1> to <4>, wherein step (C) further includes step (C') of
(C') precipitating a solid containing a polyvalent ion in the treated water.

<6> The method according to <5>, wherein the polyvalent ion is at least one selected from the group consisting of $Si^{2+}$, $Ca^{2+}$, $PO_4^{2-}$, $SO_4^{2-}$, $Mg^{2+}$, $Mn^{2+}$, $Zn^{2+}$, $Fe^{2+}$, $Fe^{3+}$, $Sr^{2+}$, $Cu^{2+}$, $Al^{3+}$, and $Sn^{3+}$.

<7> The method according to <5> or <6>, wherein step (C) further includes step (C") of
(C") removing, by sedimentation, the solid that has been precipitated in the treated water in step (C').

<8> The method according to any one of <1> to <7>, wherein, in step (D), the ion removal membrane has an $MgSO_4$ blocking rate of 60% to 100% at the time when a pressure of 3000 kPa is applied at 20°C.

<9> The method according to any one of <1> to <8>, wherein, in step (D), the ion removal membrane has a transmembrane pressure difference of 0.4 MPa to 4.14 MPa.

<10> A method for producing a polyhydroxyalkanoate, including the steps of: (a) crushing or solubilizing microorganism cells that contain a polyhydroxyalkanoate; and (b) separating the polyhydroxyalkanoate in a composition obtained in step (a), the steps (a) and (b) using recycled water produced by a method according to any one of <1> to <9>.

Examples

The following more specifically describes embodiments of the present invention on the basis of Examples. Note, however, that the present invention is not limited to these Examples.

[Measurement and evaluation methods]

The following describes measurement and evaluation methods in Examples and Comparative Examples.

(FI value)

The FI value is one of semiquantitative indexes each indicating a degree of fouling of a membrane which may be caused by influent water that is to be used in a membrane treatment. This FI value is defined in JIS K 3802. In the case of an RO membrane, it is recommended that the FI value of the influent water be set to no more than 5. According to a method for calculating the FI value, measured is a time (T1) required for 500 ml of sample water to pass through a 47 mm disc filter having a pore size of 0.45 um under 0.206 MPa. Further after a 15-minute continuous operation, measured is a time (T2) required for another 500 ml of sample water to pass through the same filter. The FI value is derived by the following formula (1).

$$FI\ value = \frac{PF}{T} = \frac{100(1 - T1/T2)}{T} \quad \cdots \ (1)$$

In the above formula (1), PF represents an occlusion factor; and T represents the time from the time when measurement of T1 starts to the time when measurement of T2 starts.

(Turbidity)

Turbidity was measured by placing 200 mL of prepared washing water in a dedicated measuring container and quietly immersing a measuring instrument (manufactured by Kasahara Chemical Instruments Corp., TCR-5Z) in the washing water so as not to cause bubbles to attach on the measuring instrument.

(Composition analysis of solid content)

Metal ions contained in a filtrate which had been subjected to pretreatment filtration by a membrane bioreactor method was analyzed with use of an ICP-MS (manufactured by Agilent Technologies, Agilent 7900). In addition, metal ions contained in a solid content adhered to a surface of the RO membrane were analyzed with use of the ICP-MS (manufactured by Agilent Technologies, Agilent 7900), and Si, P, and $SO_4$ were analyzed with use of an ICP-AES (manufactured by Shimadzu Corporation, ICPS-7510).

(Thermal stability)

The thermal stability was calculated on the basis of the weight average molecular weight retention rate of a PHA after heating at 160°C for 20 min. The thermal stability was determined to be good in a case where the weight average molecular weight retention rate was not less than 70%, whereas determined to be poor in a case where the weight average molecular weight retention rate was less than 70%. The weight average molecular weight retention rate was calculated by the following formula:

Weight average molecular weight retention rate (%) = (weight average molecular weight of PHA after heating/weight average molecular weight of PHA before heating) × 100

<Weight average molecular weight of PHA before heating>

After 10 mg of a PHA powder had been dissolved in 10 mL of chloroform, an insoluble matter was removed by filtration. A resultant solution (filtrate) was subjected to molecular weight measurement with use of chloroform as a mobile phase, by using a GPC system, manufactured by Shimadzu Corporation, to which "Shodex K805L (300 $\times$ 8 mm, two columns joined)" was attached. As a molecular weight standard sample, a commercially available standard polystyrene was used.

<Weight averaged molecular weight of PHA after heating>

A PHA sheet was prepared by performing a preheat treatment on the PHA powder for 7 minutes at 160°C and then heating the PHA powder at 160°C for 20 minutes. The weight average molecular weight of the PHA after heating was measured in a procedure similar to that in a case where the weight average molecular weight of the PHA before heating was measured, except that 10 mg of the PHA sheet was used.

(Transmembrane pressure difference)

The transmembrane pressure difference is calculated by the following formula.

$$\text{(RO membrane inlet pressure + RO membrane outlet pressure)}/2 \text{ - permeation-side (secondary-side) pressure}$$

[Example 1]

An anaerobic treatment and an aerobic treatment which were performed by microorganisms were carried out on waste water that had been discharged in production of a PHA. Subsequently, pretreatment filtration was carried out by a membrane bioreactor method with use of an MF membrane. Then, after a solid content was precipitated by adjusting pH of the filtrate, filtration with use of an RO membrane was carried out. The anaerobic treatment was carried out as follows: high molecular weight carbohydrates and lipids were degraded into organic acids and lower alcohols by the action of acidogenic bacteria in an acidogenesis tank (at a pH of approximately 7.1); and then, the organic acids and the lower alcohols were degraded into methane gas and carbon gas by the action of granular methanogens in an EGSB-type methanogenisis tank (at a loading rate of 15 kg CODcr/m3/d). The aerobic treatment was carried out by degrading, through the action of aerobic bacteria, organic substances that had not been degraded by the anaerobic treatment. The degradation was carried out in an apparatus consisting of a denitrification tank (activated sludge treatment tank), an aeration tank (activated sludge treatment tank), a second denitrification tank (activated sludge treatment tank), and a re-aeration tank (activated sludge treatment tank). Pretreatment filtration by the membrane bioreactor method was carried out by placing an MF membrane (hollow fiber membrane: PVDF, manufactured by Mitsubishi Chemical; and nominal pore size: 0.4 $\mu$m) in the re-aeration tank (activated sludge treatment tank). Water was caused to permeate the MF membrane at a filtration linear velocity of 0.35 m/day. The water that had permeated the MF membrane exhibited a pH of 7, an FI value of 0, and a turbidity of 0 NTU. The water having permeated the MF transmembrane was collected in a stirring tank, and Genesys LF (manufactured by Genesys), which was a scale inhibitor, was added to the water so as to be at a concentration of 2.4 ppm, and the water was adjusted to pH 8.8 with use of an aqueous solution of sodium hydroxide. Due to precipitation of the solid content, the FI value of water became not less than 6, and the turbidity became 0.5 NTU. This water was concentrated four-fold, so that concentrated water was prepared. Then, the solution was sent to an RO membrane (material: composite polyamide, manufactured by Nitto Denko Corporation, LFC3-LD) at a water temperature of 25°C and a filtration linear velocity of 0.48 m/day. The transmembrane pressure difference was 1.5 MPa while the solution was sent. Water that had permeated the RO membrane (also referred to as "recycled water") and water that had not permeated the RO membrane were both returned to a water collecting tank and a circulation operation was carried out. When driving was carried out at a constant filtration linear velocity, the transmembrane pressure difference increased because of fouling, and the solution was sent until the transmembrane pressure difference reached 2.0 MPa. Subsequently, as a membrane performance recovery treatment, a flushing operation was carried out for 30 minutes at a supplied water linear velocity of not less than 6 m/min with use of permeable water. After the flushing operation, concentrated water was sent at a filtration linear velocity of 0.48 m/day. This resulted in a transmembrane pressure difference of 1.4 MPa.

[Example 2]

A waste water treatment and an RO membrane recovery operation were carried out as in Example 1, except that sodium hydroxide was used to adjust the pH to 8.9. The FI value of water having a pH adjusted to 8.9 was not less than 6 and the turbidity of the water was 0.5 NTU. Then, a resultant solution was sent to the RO membrane at a water temperature of 25°C and a filtration linear velocity of 0.48 m/day. The transmembrane pressure difference at the start of sending the solution was 1.6 MPa. Sending the solution was continued until the transmembrane pressure difference reached 2.0 MPa. Thereafter, after the membrane performance recovery treatment, concentrated water was sent at a filtration linear velocity of 0.48 m/day. This resulted in a transmembrane pressure difference of 1.4 MPa.

[Example 3]

A waste water treatment and an RO membrane recovery operation were carried out as in Example 1, except that sodium hydroxide was used to adjust the pH to 9.1. The FI value of water having a pH adjusted to 9.1 was not less than 6 and the turbidity of the water was 0.6 NTU. Then, a resultant solution was sent to the RO membrane at a water temperature of 25°C and a filtration linear velocity of 0.48 m/day. The transmembrane pressure difference at the start of sending the solution was 1.4 MPa. Sending the solution was continued until the transmembrane pressure difference reached 2.0 MPa. Thereafter, after the membrane performance recovery treatment, concentrated water was sent at a filtration linear velocity of 0.48 m/day. This resulted in a transmembrane pressure difference of 1.3 MPa.

[Example 4]

A waste water treatment and an RO membrane recovery operation were carried out as in Example 1, except that the scale inhibitor was not added and sodium hydroxide was used to adjust the pH to 9.1. The FI value of water having a pH adjusted to 9.1 was not less than 6 and the turbidity of the water was 0.7 NTU. Then, a resultant solution was sent to the RO membrane at a water temperature of 25°C and a filtration linear velocity of 0.48 m/day. The transmembrane pressure difference at the start of sending the solution was 1.6 MPa. Sending the solution was continued until the transmembrane pressure difference reached 1.9 MPa. Thereafter, after the membrane performance recovery treatment, concentrated water was sent at a filtration linear velocity of 0.48 m/day. This resulted in a transmembrane pressure difference of 1.4 MPa.

[Example 5]

A waste water treatment and an RO membrane recovery operation were carried out as in Example 4, except that sodium hydroxide was used to adjust the pH to 9.2. The FI value of water having a pH adjusted to 9.2 was not less than 6 and the turbidity of the water was 0.4 NTU. Then, a resultant solution was sent to the RO membrane at a water temperature of 25°C and a filtration linear velocity of 0.48 m/day. The transmembrane pressure difference at the start of sending the solution was 1.4 MPa. Sending the solution was continued until the transmembrane pressure difference reached 1.9 MPa. Thereafter, after the membrane performance recovery treatment, concentrated water was sent at a filtration linear velocity of 0.48 m/day. This resulted in a transmembrane pressure difference of 1.5 MPa.

[Example 6]

A waste water treatment and an RO membrane recovery operation were carried out as in Example 4, except that sodium hydroxide was used to adjust the pH to 9.2. The FI value of water having a pH adjusted to 9.2 was not less than 6 and the turbidity of the water was 0.3 NTU. Then, a resultant solution was sent to the RO membrane at a water temperature of 25°C and a filtration linear velocity of 0.48 m/day. The transmembrane pressure difference at the start of sending the solution was 1.5 MPa. Sending the solution was continued until the transmembrane pressure difference reached 2.0 MPa. Thereafter, after the membrane performance recovery treatment, concentrated water was sent at a filtration linear velocity of 0.48 m/day. This resulted in a transmembrane pressure difference of 1.6 MPa.

[Comparative Example 1]

A waste water treatment and an RO membrane recovery operation were carried out as in Example 1, except that pretreatment filtration was carried out by the membrane bioreactor method with use of an MF membrane but no alkali treatment was carried out after the pretreatment filtration. The FI value of resultant water was 0; the turbidity of the water was 0 NTU. Then, a resultant solution was sent to the RO membrane at a water temperature of 25°C and a filtration linear velocity of 0.48 m/day. The transmembrane pressure difference at the start of sending the solution was 1.3 MPa. Sending the solution was continued until the transmembrane pressure difference reached 2.3 MPa. Thereafter, after the membrane performance recovery treatment, concentrated water was sent at a filtration linear velocity of 0.48 m/day. This resulted in a transmembrane pressure difference of 2.2 MPa.

(Results)

Table 1 shows the results of measurements of the initial transmembrane pressure difference, the transmembrane difference at the end of filtration, and the transmembrane pressure difference after the membrane performance recovery treatment for Examples 1 to 6 and for Comparison Example 1.

[Table 1]

| | Transmembrane pressure difference at start of sending solution [MPa] | Transmembrane pressure difference at end of sending solution [MPa] | Transmembrane pressure difference after membrane performance recovery treatment [MPa] |
|---|---|---|---|
| Example 1 | 1.5 | 2 | 1.4 |
| Example 2 | 1.6 | 2 | 1.4 |
| Example 3 | 1.4 | 2 | 1.3 |
| Example 4 | 1.6 | 1.9 | 1.4 |
| Example 5 | 1.4 | 1.9 | 1.5 |
| Example 6 | 1.5 | 1.9 | 1.6 |
| Comparative Example 1 | 1.3 | 2.3 | 2.2 |

It is clear from Table 1 that Examples 1 to 6 each have a smaller transmembrane pressure difference after the membrane performance recovery treatment than Comparative Example 1. Since there was no significant difference in the transmembrane pressure difference after the end of sending the solution, it was shown that the flux rate of the RO membrane could be recovered to a high value by the membrane performance recovery treatment. The results of Examples 1 and 4 and Comparative Example 1 suggested that precipitation of the solid content, but not the scale inhibitor, contributed to membrane recovery.

Table 2 shows the metal ion concentration of the filtrate obtained as a result of pretreatment filtration carried out by the membrane bioreactor method in Example 6. Further, Table 3 shows the type and concentration of the metal ions of the solid content adhered to the RO membrane after the end of sending the solution in Example 6.

[Table 2]

| Metal ion | Concentration after pretreatment filtration [mg/L] |
|---|---|
| Na | 795 |
| K | 42 |
| Ca | 11 |
| Mg | 7 |
| Fe | 0.17 |
| Cu | 0.06 |
| Al | 0.1 |
| Mn | 0.04 |
| Sr | less than 0.1 |
| Zn | less than 0.05 |
| Ba | less than 0.01 |
| Co | less than 0.01 |

[Table 3]

| Metal ion | Concentration of metal ions adhered to RO membrane [wt%] |
|---|---|
| Si | 10 |
| Ca | 10 |
| Na | 6 |
| Mg | 2 |
| Mn | 0.4 |
| K | 0.3 |
| Zn | 0.1 |
| Fe | 0.06 |
| Sr | 0.06 |
| Cu | 0.02 |
| Al | 0.01 |
| Sn | 0.007 |
| P | 8 |
| $SO_4$ | 5 |

It is found that as compared with the results in Table 2, in the results in Table 3, the proportion of elements each of which becomes a polyvalent ion is higher. Therefore, it can be inferred that pH adjustment by the alkali treatment resulted in preferential precipitation of polyvalent ions having a low solubility. Therefore, it is considered that the membrane performance could be restored by carrying out the membrane performance recovery treatment with permeable water, without firm adhesion of the metal ions to the RO membrane.

On the other hand, in the comparative example, similar metal salts were adhered to a similar RO membrane surface (data not shown). However, it is inferred that in the comparative example, metal ions were condensed and precipitated on the surface of the RO membrane. It is considered that for the above reason, the metal salts had firmly adhered to the RO membrane surface and even when the membrane performance recovery treatment with use of permeable water was carried out, the membrane performance could not be recovered.

[Example 7]

(Preparation of bacteria culture fluid)

Bacteria were cultured in a manner described in International Publication No. WO 2010/067543, and a bacteria culture fluid containing bacteria which contain PHA was obtained. Note that at present, Ralstonia utropha is classified as Capriavidus necator. The composition ratio of repeating units of the PHA (composition ratio of 3 HB units/3 HH units) contained in the above bacteria was 92/8 to 99/ 1 (mol/mol).

(Sterilization treatment)

The bacteria culture fluid obtained above was subjected to a sterilization treatment by heating and stirring at an internal temperature of 60°C to 80°C for 20 minutes.

(Purification treatment)

Sodium dodecyl sulfate was added so as to be at 0.2% by weight, to the bacteria culture fluid which had been obtained above and sterilized. In addition, the washing water in which sodium hydroxide was dissolved was added so that the pH became 11.0, and then, the bacteria culture fluid was kept at 50°C for 1 hour. Subsequently, high-pressure crushing was carried out at a pressure of 44 MPa to 54 MPa with use of a high-pressure crusher (high-pressure homogenizer model PA2K type manufactured by Niro Soavi S.p.A.).

To a crushed bacteria solution which had been obtained above after the high-pressure crushing, the washing water was added in a weight that was the same as the weight of the crushed bacteria solution. After centrifugation of a resultant solution thus obtained, supernatant was removed, so that the solution was concentrated two-fold. To this concentrated aqueous suspension of the PHA, the washing water (pH 11.0) to which sodium hydroxide had been added was added in a weight which was the same as the weight of the supernatant that had been removed, and then centrifugation was carried out. Next, after supernatant was removed, the washing water was added so that a residual after removal of the supernatant was suspended in the washing water. Then, 0.2% by weight sodium dodecyl sulfate, and protease (manufactured by Novozymes A/S; Esperase) whose amount was 1/100 by weight of the PHA were added. Then, a resultant suspension was stirred for 2 hours, while being maintained at 50°C and at pH 10.0. Subsequently, supernatant was removed by centrifugation and the suspension was concentrated fivefold. To this concentrated aqueous suspension of the PHA, the washing water (pH 11.0) to which sodium hydroxide had been added was added in a weight which was the same as the weight of the supernatant which had been removed, and then centrifugation was carried out. After the same operation was repeated five times, supernatant was removed, so that a PHA aqueous suspension whose PHA concentration was adjusted to 52% by weight was obtained. In this purification treatment step, as the washing water, water (recycled water) which had been obtained by causing the concentrated water of Example 1 to permeate the RO membrane was used.

(Granulation)

To the PHA aqueous suspension (solid content concentration: 52% by weight) obtained above, a dispersing agent (polyethylene glycol-polypropylene glycol-block ether type nonionic surfactant, product name "PLONON #208", manufactured by NOF CORPORATION) was added in an amount of 1 pHr (1 part by weight relative to 100 parts by weight of the PHA present in the aqueous suspension). Thereafter, the solid content concentration was adjusted to 30% by weight with distilled water. After this solution was stirred for 30 minutes, sulfuric acid was added and the pH was adjusted until the pH was stabilized at 4. A resultant PHA aqueous suspension was dried at 60°C for 12 hours, so that a PHA powder was obtained.

[Comparative Example 2]

Washing water 2 was obtained by treating industrial water (produced by Kaneka) with an ion exchange resin (manufactured by Organo Corporation, a strongly acidic cation resin and a strongly basic anion resin). In the purification treatment step, the PHA powder was obtained as in Example 7, except that the washing water 2 was used as the washing water.
Thermal stability of the PHA powder obtained in each of Example 7 and Comparative Example 2 was measured and evaluated as described above. Table 4 shows evaluation results.

[Table 4]

|  | Thermal stability [%] |
|---|---|
| Example 7 | 81 |
| Comparative Example 2 | 76 |

(Results)

It is clear from Table 4 that the PHA powder of Example 7 had better thermal stability than the PHA powder of Comparative Example 2. Therefore, it has been shown that use of the recycled water in accordance with an embodiment of the present invention makes it possible to produce a PHA that has a better performance (improved thermal stability) than use of conventional washing water.

Industrial Applicability

The present invention can be preferably utilized in the field of waste water treatment and in other fields.

**Claims**

**1.** A method for producing recycled water, the method comprising the following steps (A) to (D) of:

(A) subjecting waste water to an anaerobic treatment and an aerobic treatment which are carried out by microorganisms, the waste water having been discharged during a production process of a polyhydroxyalkanoate;
(B) subjecting treated water obtained in step (A) to pretreatment filtration performed by a membrane bioreactor method;
(C) subjecting the treated water obtained in step (B) to an alkali treatment; and
(D) filtering, through an ion removal membrane, the treated water obtained in step (C).

2. The method according to claim 1, wherein in step (C), pH of the treated water is adjusted to 7 to 11, turbidity of the treated water is adjusted to not less than 0.1, and FI value of the treated water is adjusted to not less than 4.5.

3. The method according to claim 1 or 2, wherein in step (C), FI value of the treated water is adjusted to not less than 6.0.

4. The method according to any one of claims 1 to 3, wherein the alkali treatment is carried out with use of at least one selected from the group consisting of an aqueous solution of an alkali metal hydroxide, an aqueous solution of an alkali metal carbonate, an aqueous solution of an alkali metal hydrogen carbonate, an aqueous solution of organic acid alkali metal, an aqueous solution of an alkali metal borate, an aqueous solution of an alkali metal phosphate, an aqueous solution of an alkali earth metal hydroxide, and ammonia water.

5. The method according to any one of claims 1 to 4, wherein step (C) further includes step (C') of
(C') precipitating a solid containing a polyvalent ion in the treated water.

6. The method according to claim 5, wherein the polyvalent ion is at least one selected from the group consisting of $Si^{2+}$, $Ca^{2+}$, $PO_4^{2-}$, $SO_4^{2-}$, $Mg^{2+}$, $Mn^{2+}$, $Zn^{2+}$, $Fe^{2+}$, $Fe^{3+}$, $Sr^{2+}$, $Cu^{2+}$, $Al^{3+}$, and $Sn^{3+}$.

7. The method according to claim 5 or 6, wherein step (C) further includes step (C") of
(C") removing, by sedimentation, the solid that has been precipitated in the treated water in step (C').

8. The method according to any one of claims 1 to 5, wherein, in step (D), the ion removal membrane has an $MgSO_4$ blocking rate of 60% to 100% at the time when a pressure of 3000 kPa is applied at 20°C.

9. The method according to any one of claims 1 to 8, wherein, in step (D), the ion removal membrane has a transmembrane pressure difference of 0.4 MPa to 4.14 MPa.

10. A method for producing a polyhydroxyalkanoate, comprising the steps of:

(a) crushing or solubilizing microorganism cells that contain a polyhydroxyalkanoate; and
(b) separating the polyhydroxyalkanoate in a composition obtained in step (a),
the steps (a) and (b) using recycled water produced by a method according to any one of claims 1 to 9.

## INTERNATIONAL SEARCH REPORT

International application No.

**PCT/JP2022/003969**

### A. CLASSIFICATION OF SUBJECT MATTER

***C02F 1/44***(2006.01)i; ***B01D 61/02***(2006.01)i; ***B01D 61/14***(2006.01)i; ***B01D 61/58***(2006.01)i; ***B01D 65/08***(2006.01)i;
***C02F 1/58***(2006.01)i; ***C02F 1/60***(2006.01)i; ***C02F 1/62***(2006.01)i; ***C02F 1/64***(2006.01)i; ***C02F 3/12***(2006.01)i;
***C02F 3/30***(2006.01)i; ***C02F 3/34***(2006.01)i; ***C12P 1/00***(2006.01)i; ***C12P 7/62***(2022.01)i
FI: C02F1/44 C; C12P7/62; C12P1/00 Z; B01D61/02; B01D61/14 500; B01D65/08; C02F1/44 F; C02F1/58 J; C02F1/58 K; C02F1/58 Q; C02F1/58 R; C02F1/60; C02F1/62; C02F1/64; C02F3/30 Z; B01D61/58; C02F3/12 S; C02F3/12 V; C02F3/34 101A

According to International Patent Classification (IPC) or to both national classification and IPC

### B. FIELDS SEARCHED

Minimum documentation searched (classification system followed by classification symbols)

C02F1/44; B01D61/02; B01D61/14; B01D61/58; B01D65/08; C02F1/58; C02F1/60; C02F1/62; C02F1/64; C02F3/12; C02F3/30; C02F3/34; C12P1/00; C12P7/62

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| A | JP 2005-254207 A (NISHIHARA CO., LTD.) 22 September 2005 (2005-09-22) | 1-10 |
| A | WO 2017/221755 A1 (KANEKA CORP.) 28 December 2017 (2017-12-28) | 1-10 |
| A | JP 2006-43611 A (KURITA WATER INDUSTRIES LTD.) 16 February 2006 (2006-02-16) | 1-10 |
| A | JP 2019-136652 A (ORGANO CORP.) 22 August 2019 (2019-08-22) | 1-10 |

☐ Further documents are listed in the continuation of Box C.  ☑ See patent family annex.

| | |
|---|---|
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| **25 March 2022** | **05 April 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

INTERNATIONAL SEARCH REPORT
Information on patent family members

International application No.

**PCT/JP2022/003969**

| Patent document cited in search report | | | Publication date (day/month/year) | Patent family member(s) | | | Publication date (day/month/year) |
|---|---|---|---|---|---|---|---|
| JP | 2005-254207 | A | 22 September 2005 | (Family: none) | | | |
| WO | 2017/221755 | A1 | 28 December 2017 | US | 2019/0330089 | A1 | |
| | | | | EP | 3476943 | A1 | |
| | | | | CN | 109312372 | A | |
| JP | 2006-43611 | A | 16 February 2006 | (Family: none) | | | |
| JP | 2019-136652 | A | 22 August 2019 | US | 2021/0061691 | A1 | |
| | | | | WO | 2019/156144 | A1 | |
| | | | | CN | 111670168 | A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 7031489 A
- JP 2008193940 A
- JP 5093049 A
- WO 2008010296 A
- WO 2010067543 A